# EUROPÄISCHE PATENTSCHRIFT

(11) **EP 2 622 353 B1**
(45) Veröffentlichungstag und Bekanntmachung des Hinweises auf die Patenterteilung: **15.11.2017**
(21) Anmeldenummer: 11767628.8
(22) Anmeldetag: 28.09.2011
(51) Int. Cl.: G01N 33/574, G01N 33/68

(54) **DIFFERENZIALDIAGNOSTIK VON PANKREASADENOMEN**
DIFFERENTIAL DIAGNOSIS OF PANCREATIC ADENOMAS
DIAGNOSTIC DIFFÉRENTIEL D'ADÉNOMES DU PANCRÉAS

(30) Priorität: 01.10.2010 DE 102010047069
(43) Veröffentlichungstag der Anmeldung: 07.08.2013
(73) Patentinhaber: Universität zu Lübeck, 23538 Lübeck (DE)
(72) Erfinder: HABERMANN, Jens, 29227 Celle (DE); GEMOLL, Timo, 23775 Großenbrode (DE); ROBLICK, Uwe, Johannes, 23552 Lübeck (DE)
(74) Vertreter: Steinecke, Peter
(86) Internationale Anmeldenummer: PCT/EP2011/004847
(87) Internationale Veröffentlichungsnummer: WO 2012/041494

(56) Entgegenhaltungen:
- WO-A1-2008/032084
- WO-A1-2011/025542
- WO-A2-2004/097052
- DE-A1-102005 061 655
- GNJATIC, S., RITTER, E., BUCHLER, M. W., GIESE, N. A., BRORS, B., FREI, C., MURRAY, A., HALAMA, N., ZORNIG, I., CHEN, Y. T.: "Seromic profiling of ovarian and pancreatic cancer", PROC NATL ACAD SCI U S A, Bd. 107, 16. März 2010 (2010-03-16), Seiten 5088-5093, XP002664707,
- WESTMORELAND J J ET AL: "Pdk1 activity controls proliferation, survival, and growth of developing pancreatic cells", DEVELOPMENTAL BIOLOGY, ACADEMIC PRESS, NEW YORK, NY, US, Bd. 334, Nr. 1, 1. Oktober 2009 (2009-10-01), Seiten 285-298, XP026600684, ISSN: 0012-1606, DOI: 10.1016/J.YDBIO.2009.07.030 [gefunden am 2009-07-25]
- KOTERA Y ET AL: "HUMORAL IMMUNITY AGAINST A TANDEM REPEAT EPITOPE OF HUMAN MUCIN MUC-1 IN SERA FROM BREAST, PANCREATIC, AND COLON CANCER PATIENTS", CANCER RESEARCH, AMERICAN ASSOCIATION FOR CANCER RESEARCH, US, vol. 54, 1 June 1994 (1994-06-01), pages 2856-2860, XP000764847, ISSN: 0008-5472
- MISEK DAVID E ET AL: "Early detection and biomarkers in pancreatic cancer.", JOURNAL OF THE NATIONAL COMPREHENSIVE CANCER NETWORK : JNCCN NOV 2007, vol. 5, no. 10, November 2007 (2007-11), pages 1034-1041, ISSN: 1540-1405
- JANE TREPEL ET AL: "Targeting the dynamic HSP90 complex in cancer", NATURE REVIEWS. CANCER, NATUR PUBLISHING GROUP, LONDON, GB, vol. 10, no. 8, 1 August 2010 (2010-08-01) , pages 537-549, XP002687187, ISSN: 1474-175X, DOI: 10.1038/NRC2887
- ANGELA L. MCCLEARY-WHEELER ET AL: "Insights into the epigenetic mechanisms controlling pancreatic carcinogenesis", CANCER LETTERS, vol. 328, no. 2, 1 January 2013 (2013-01-01), pages 212-221, XP55201879, ISSN: 0304-3835, DOI: 10.1016/j.canlet.2012.10.005

## Beschreibung

Die Erfindung betrifft ein Verfahren zur *in vitro* Diagnose bei Pankreasadenomen, die Verwendung von Biomarkern in diesem Verfahren, die Verwendung von Kits zum Durchführen des Verfahrens und die Verwendung dieses Verfahrens zum Nachweis von Pankreasadenomen und zur Beurteilung des Fortschreitens zum Pankreaskrebs.

Das duktale Adenokarzinom des Pankreas ist in den westlichen Industrieländern eine Tumorerkrankung mit steigender Inzidenz und liegt mit einer 5-Jahres Überlebensrate von 1 % an 4.-5. Stelle der tumorbedingten Todesursachen (Gudjonsson, B. (1987) Cancer of the pancreas. 50 years of surgery. Cancer 60, 2284-2303; Parker, S. L., Tong, T., Bolden, S., and Wingo, P. A. (1997) Cancer statistics, 1997. CA Cancer J Clin 47, 5-27). Männer sind häufiger betroffen als Frauen und 2/3 aller Patienten haben bei Diagnosestellung das 60. Lebensjahr bereits überschritten (Urrutia, R. (1997) Exploring the role of homeobox and zinc finger proteins in pancreatic cell proliferation, differentiation, and apoptosis. Int J Pancreatol 22, 1-14). Die Kombination aus früh einsetzender Metastasierung und oftmals nur unzureichenden Diagnose- und Therapiemöglichkeiten sind die Hauptursachen für die besonders ungünstige Prognose. Da zum Zeitpunkt der Diagnose in bis zu 50 % der Patienten Lymphknoten und periphere Nerven von den Tumorzellen befallen sind (Takahashi, T., Ishikura, H., Motohara, T., Okushiba, S., Dohke, M., and Katoh, H. (1997) Perineural invasion by ductal adenocarcinoma of the pancreas. J Surg Oncol 65, 164-170), können nur noch 10 - 20 % aller Patienten durch radikale Resektion des Primärtumors behandelt werden (Zamboni, G., Capelli, P., Pesci, A., Beghelli, S., Luttges, J., and Kloppel, G. (2000) Pancreatic head mass: what can be done? Classification: the pathological point of view. JOP 1, 77-84). Routinemäßige klinische Diagnostik umfasst körperliche Untersuchungen, Sonographie und Computertomographie. Bringen Computertomogramm und Ultraschall keine ausreichende Information, so können als weitere bildgebende Verfahren die Kernspintomographie oder, seltener, die Positronen-Emissions-Tomographie zum Einsatz kommen. Eine eindeutige Diagnosesicherung ist heutzutage allerdings nur durch die Untersuchung von Gewebe unter dem Mikroskop möglich. Dieses Gewebe wird mit Hilfe der Endoskopie, Endosonographie, Ultraschalluntersuchung oder der Computertomographie durch Punktion durch die Haut, dann meist unter örtlicher Betäubung, gewonnen. Alle Methoden zur Diagnostik eines Pankreaskarzinoms werden zumeist erst dann durchgeführt, wenn bereits Symptome bestehen, die ein fortgeschrittenes Krankheitsstadium anzeigen.

Bisher ist es also nicht möglich, präoperativ eine Unterscheidung von gutartigen Pankreasveränderungen, z.B. Pankreasadenomen, und Pankreaskarzinomen aufgrund der Routinediagnostik vorzunehmen. Eine Differenzierung würde den operativen Eingriff entsprechend detaillierter planen lassen: prophylaktisch würde das Pankreasadenom operativ entfernt werden. Das Vorgehen würde sich dabei aber nicht onkologischen Richtlinien unterwerfen müssen, d.h., dass im günstigsten Falle Teile des Pankreas sogar erhalten bleiben könnten, der Eingriff weniger aggressiv wäre, und die Patienten dadurch eine kürzere Rekonvaleszenzzeit hätten.

Aus dem Stand der Technik sind Proteinmarker bekannt, die mit der Detektion von Pankreaskarzinomen bzw. Pankreasadenomen in Verbindung gebracht werden können. PDPK1/PDK1 (3-Phosphoinositide Dependent Protein Kinase 1) gilt als relevant für die Entwicklung von Pankreastumoren. PDK1 wird z.B. als Zellwachstumsregulator in Pankreaszellen und als eventuellen Aktivator von mTORC2 in exokrinen Zellen beschrieben (Westmoreland et al. (2009) Pdk1 activity controls proliferation, survival, and growth of developing pancreatic cells. Developmental Biology 334, 285 -298). Die DE 10 2005 061655 A1 beschreibt die Verwendung von Inhibitoren von PDK1 zur Herstellung eines Medikaments zur Prophylaxe oder Behandlung von Krebs, unter anderem auch von Pankreaskrebs. Die WO 2004/097052 legt Expressionsprofile bestimmter Gene in PBMCs (peripheral blood mononuclear cells) von Krebspatienten dar, unter anderem auch von CHTOP (Chromatin Target Of PRMT1).

Keine der zitierten Quellen behandelt die Fragestellung der Immunantwort von Patienten gegen Krebs und die Detektion bestimmter Antigene im Serum. Es werden lediglich Gewebeproteine bzw. unterschiedlich exprimierte Gene beschrieben, die zusätzlich nicht mit einer frühen Detektion von Pankreaskarzinomen bzw. Pankreasadenomen in Verbindung gebracht werden. Zwischen Gen- und Protein/Antigen-Regulation spielen im Organismus diverse regulatorische Vorgänge (z.B. Post-Translationale Modifikationen und Epigentik) eine Rolle, die es nicht ermöglichen, Expressionsunterschiede zwischen DNA- und Proteinebenen - insbesondere im Serum - vorherzusagen (siehe z.B. McClearly-Wheeler et al. (2013) Cancer Letter 328, 212-221 und Trepel et al. (2010) Nature Reviews Cancer 10, 537-549).

Es gibt eine Fülle von Beweisen für die Existenz einer Immunantwort gegen Krebs beim Menschen, welche durch das Vorhandensein von Autoantikörpern in Seren von Krebspatienten gezeigt werden konnte. Autoantigene verändern sich dabei vor oder während der Tumorbildung und können nachfolgend eine Immunreaktion über Autoantikörper auslösen (Chatterjee, M., et al. (2006) Diagnostic markers of ovarian cancer by highthroughput antigen cloning and detection on arrays. Cancer Res 66, 1181-1190; Hudson, M. E., Pozdnyakova, I., Haines, K., Mor, G., and Snyder, M. (2007) Identification of differentially expressed proteins in ovarian cancer using high-density protein microarrays. Proc Natl Acad Sci U S A 104, 17494-17499; Soussi, T. (2000) p53 Antibodies in the sera of patients with various types of cancer: a review. Cancer Res 60, 1777-1788).

Die WO 2008/032084 A1 beschreibt ein Verfahren zur Diagnose, Prognose und Überwachung von Krebs über den Nachweis unter anderem von Autoantikörpern gegen Tumormarkerproteinen. Die WO 2011/025542 A1 beschreibt Verfahren, Zusammensetzungen und Kits für die Diagnose, Prognose und Behandlung von Krebs, insbesondere von Ovarialkarzinomen, anhand von Autoantikörpern.

Auch beim Pankreaskarzinom wurden schon Untersuchungen zu Autoantikörpern vollzogen. So wurden zweidimensionale Gelelektrophorese, Western Blots und Massenspektrometrie zur Identifizierung von Antikörpern gegen Pankreaskrebs und Pankreatitis verwendet (Hong, S. H., Misek, D. E., Wang, H., Puravs, E., Giordano, T. J., Greenson, J. K., Brenner, D. E., Simeone, D. M., Logsdon, C. D., and Hanash, S. M. (2004) An autoantibody-mediated immune response to calreticulin isoforms in pancreatic cancer. Cancer Res 64, 5504-5510). Isoformen von Calreticulin wurden hierbei als mögliche Marker zur frühen Diagnose beschrieben. Dieser Ansatz erlaubt, im Gegensatz zum Proteinarray, die Analyse von Proteinen in ihrem natürlichen Zustand und Umfeld. Allerdings ist man auf die Auflösung der zweidimensionalen Gelelektrophorese von ca. 1500 Proteinen beschränkt.

Auch Antikörpermicroarrays wurden bereits erfolgreich in der Pankreaskrebsforschung eingesetzt. Solche Microarrays ermöglichen die Erkennung von spezifischen Serum-Autoantikörpern an einer sehr großen Anzahl von Targets gleichzeitig. Die Arrays können mit hohem Durchsatz eingesetzt werden, um Muster von durch Autoantikörper erkannten Antigenen im Verlauf der Krankheit zu bestimmen. Gnjatic *et al.* verwendeten die Version v4.0 des Protoarrays von Invitrogen (ca. 8000 Targets) zum Vergleich von Pankreaskrebspatienten mit einer Kontrollgruppe (n = 113). Es wurden 29 Serumproteine identifiziert, die als Biomarker zur Klassifikation bestimmter Pankreastumorstadien dienen könnten (Gnjatic, S., Ritter, E., Buchler, M. W., Giese, N. A., Brors, B., Frei, C., Murray, A., Halama, N., Zornig, I., Chen, Y. T., Andrews, C., Ritter, G., Old, L. J., Odunsi, K., and Jager, D. (2010) Seromic profiling of ovarian and pancreatic cancer. Proc Natl Acad Sci U S A 107, 5088-5093). Die Bestimmung von Autoantikörpern gegen einzelne Proteine zur Diagnose von Pankreaskrebs wird ebenfalls von Kotera et al. ((1994) Cancer Res. 54, 2856-60) und Misek et al. ((2007) J Natl Compr Canc Netw. 5, 1034-41) beschrieben.

Detaillierte Untersuchungen an humanen Gewebeproben sowie neue Mausmodelle haben die frühe Pankreastumorentwicklung von den Vorläuferläsionen bis hin zum invasiven Karzinom aufzeigen können (Kleeff, J., Michalski, C. W. and Friess H. (2008) Molekulare Aspekte des Pankreaskarzinoms - ein Update. Onkopipeline 1, 57-62). Bisher wurden allerdings keine tumorspezifischen Autoantikörper identifiziert, welche charakteristisch für Frühstadien von Pankreastumoren insbesondere Pankreasadenomen sind und so als potentielle Biomarker zur Differenzialdiagnose dienen können. *In vitro* Screeningtests, die eine Unterscheidung zwischen Pankreasadenomen und Pankreaskarzinomen ermöglichen gibt es also derzeit nicht. Dem zufolge besteht Bedarf an innovativen Screening- und Therapie-Methoden, welche zu einer früheren Diagnose und einer deutlichen Prognoseverbesserung von Pankreaskrebspatienten führen können.

Der vorliegenden Erfindung liegt damit die Aufgabe zugrunde, ein Verfahren zur *in vitro* Diagnose von Pankreasadenom und zur Unterscheidung zwischen Pankreasadenom und Pankreaskarzinom bereitzustellen.

Die Aufgabe wird gelöst durch ein Verfahren zur *in vitro* Diagnose eines Pankreasadenoms, mit den Schritten:
(a) Inkubieren einer einem Individuum entnommenen Antikörper enthaltenden Probe mit wenigstens einem von einem der Gene ausgewählt aus einer ersten Gruppe von Genen bestehend aus KCNAB1, C1ORF77, SPSB2, MED9, STK40, ITGB3BP, SCEL, PDPK1, DUSP15 und MED 19 codierten Protein oder Proteinfragments,
(b) Bestimmen der an das wenigstens eine Protein oder das Proteinfragment gebundenen Antikörpermenge; und
(c) Vergleichen der aus der Probe an das wenigstens eine Protein oder Proteinfragment gebundenen Antikörpermenge mit der bei gesunden Individuen im Durchschnitt gegen das wenigstens eine Protein oder Proteinfragment vorliegenden Antikörpermenge,
wobei:
- eine niedrigere Menge des bestimmten gegen das wenigstens eine von der ersten Gruppe von Genen codierte Protein oder Proteinfragment gerichteten Antikörpers
   das Vorliegen eines Pankreasadenoms anzeigt.

In einer Ausführungsform umfasst das Verfahren weiterhin die Schritte:
(a) Inkubieren der Probe mit wenigstens einem von einem der Gene ausgewählt aus einer zweiten Gruppe von Genen bestehend aus NAP1L3, TMOD3, CSPP1, TMOD2, INPP5A und IGHG1 codierten Protein oder Proteinfragments,
(b) Bestimmen der an das wenigstens eine Protein oder das Proteinfragment gebundenen Antikörpermenge; und
(c) Vergleichen der aus der Probe an das wenigstens eine Protein oder Proteinfragment gebundenen Antikörpermenge mit der bei gesunden Individuen im Durchschnitt gegen das die wenigstens eine Protein oder Proteinfragment vorliegenden Antikörpermenge,
wobei eine höhere Menge des bestimmten gegen das wenigstens eine von der zweiten Gruppe von Genen codierte Protein oder Proteinfragment gerichteten Antikörpers, das Vorliegen eines Pankreasadenoms anzeigt.

In einer Ausführungsform ist das wenigstens eine Protein oder Proteinfragment von TMOD3 oder INPP5A codiert.

In einer Ausführungsform ist die Probe Blut oder Serum.

In einer Ausführungsform ist das wenigstens eine Protein oder Proteinfragment an einen Träger gebunden.

In einer Ausführungsform erfolgt das Bestimmen der an das wenigstens eine Protein oder Proteinfragment gebundenen Antikörpermenge mittels markierter anti-human-Immunglobulin-Antikörper.

In einer Ausführungsform sind die anti-human-Immunglobulin-Antikörper mit einem Enzym, Biotin, einem radioaktiven Isotop oder einem Fluoreszenzfarbstoff markiert.

Die Aufgabe der vorliegenden Erfindung wird außerdem durch die Verwendung eines Kits zum Durchführen des oben beschriebenen Verfahrens gelöst,
gekennzeichnet durch
wenigstens ein von einem der Gene ausgewählt aus einer ersten Gruppe von Genen bestehend aus KCNAB1, C1ORF77, SPSB2, MED9, STK40, ITGB3BP, SCEL, PDPK1, DUSP15 und MED19 und gegebenenfalls wenigstens einem von einem der Gene ausgewählt aus einer zweiten Gruppe von Genen bestehend aus NAP1L3, TMOD3, CSPP1, TMOD2, INPP5A und IGHG1 codierten Protein oder Proteinfragment; und
wenigstens einen markierten anti-human-Immunglobulin-Antikörper.

In einer Ausführungsform ist das wenigstens eine Protein oder Proteinfragment von TMOD3 oder INPP5A codiert.

In einer Ausführungsform ist das Protein oder Proteinfragment an einem Träger immobilisiert.

In einer Ausführungsform ist der anti-human-Immunglobulin-Antikörper mit einem Enzym, Biotin, einem radioaktiven Isotop oder einem Fluoreszenzfarbstoff markiert.

Die Aufgabe der vorliegenden Erfindung wird auch gelöst durch die Verwendung wenigstens eines Autoantikörpers gerichtet gegen ein Protein oder Proteinfragment, das von einem Gen ausgewählt aus der Gruppe bestehend aus KCNAB1, C1ORF77, SPSB2, MED9, STK40, ITGB3BP, SCEL, PDPK1, DUSP15 und MED19 codiert ist, als diagnostischer Marker für die Diagnose eines Pankreasadenoms.

Die Aufgabe wird gelöst durch ein Verfahren zur *in vitro* Diagnose eines Pankreasadenoms, wobei die Menge an Autoantikörper gegen mindestens eines der von den in Tabelle 1 und/oder Tabelle 2 aufgeführten Genen kodierten Proteinen in einer Probe biologischen Ursprungs, insbesondere im Serum eines Individuums bestimmt wird. Die Menge an Autoantikörper in dieser Probe wird mit der Menge des entsprechenden Autoantikörpers, die in gesunden Patienten üblicherweise vorliegt, verglichen. Falls es sich um ein in der Tabelle 1 aufgeführtes Gen handelt, ist eine erniedrigte Menge des Autoantikörpers
ein Indiz für das Vorliegen eines Pankreasadenoms. Falls es sich um ein in der Tabelle 2 aufgeführtes Gen handelt, ist eine erhöhte Menge des Autoantikörpers ein Indiz für das Vorliegen eines Pankreasadenoms.

Der Erfindung liegt eine Untersuchung zur Identifizierung von Autoantikörpern und Tumorassoziierten Antigenen bei Pankreasadenomen als Vorläufer von Karzinomen zugrunde, in der ermittelt wurde, welche Autoantikörper im Pankreasadenom eine veränderte Höhe des Vorkommens gegenüber gesunden Patienten aufweisen. Dazu wurde eine vergleichende Untersuchung von Serumproben von Patienten mit Pankreasadenomen (n = 5), Pankreaskarzinomen (n = 10) und von gesunden Patienten (n = 10) mittels High-Density Protein-Microarrays (Invitrogen, v5.0; > 9000 menschliche Proteine) durchgeführt (Beispiel 1). Beim Vergleich der erhaltenen Profile wurden insgesamt 17 Proteine statistisch signifikant zwischen Pankreasadenom und gesunden Kontrollen detektiert (p < 0,05; Tabellen 1 und 2). Dabei zeigten 7 Antikörper eine Hoch- (Tabelle 2) und 10 eine Herunterregulation (Tabelle 1) in Pankreasadenomen. Autoantikörper, die einen signifikanten Unterschied im Vergleich zwischen Pankreaskarzinomen und gesunden Kontrollen (n = 9) sowie im Vergleich zwischen Pankreasadenomen und -karzinomen (n = 9) zeigten, wurden vom Ergebnis ausgeschlossen.

Auf diese Weise wurden ein Pankreas-assoziiertes Autoantikörper-Repertoire und entsprechende Tumor-assoziierte Antigene identifiziert. Das veränderte Vorkommen dieser Proteine ist ursächlich mit dem Pankreastumor verbunden. Im Vergleich zu früheren Studien wurde mit der neusten Version des Protoarrays der Firma Invitrogen eine Vielzahl an neuen Proteinen detektiert, die als potentielle Biomarker für eine frühe Detektion des Pankreaskarzinoms dienen könnten. Bekräftigt wurde dies durch eine spezielle Patientenkohorte, die die Karzinogenese von gesunden Individuen über Pankreasadenome bis hin zum Pankreaskarzinom widerspiegelte.

Signifikante Expressionsunterschiede zwischen Pankreasadenomen und gesunden Kontrollen konnten für zwei Proteine (TMOD3 und INPP5A) auch noch mit Hilfe eines unabhängigen statistischen Verfahrens bestätigt werden. Hierbei wurden die quantifizierten Signale der ProtoArrays vom Hintergrund subtrahiert, normalisiert und log2 transformiert. Der Mann Whitney U Test diente zur Detektion unterschiedlich regulierter Targets.

TMOD3 und INPP5A wurden also durch zwei unterschiedliche statistische Verfahren ermittelt und scheinen somit besonders gut geeignet zu sein, Pankreasadenome von gesunden Kontrollen zu unterscheiden.

Überraschenderweise war von keinem der 17 gefundenen Proteinen bekannt, dass sie im Zusammenhang mit Pankreastumor stehen. Somit wird das Vorhandensein von spezifischen Antikörpern für Pankreastumoren und insbesondere für Pankreasadenome bestätigt. Das neue individuelle Muster von Proteinen ermöglicht, Pankreaskarzinome mit höherer Spezifität und Sensitivität als die bisher bekannten Serum-Biomarker in frühen Phasen der Karzinomentstehung zu detektieren und zu diagnostizieren. Dadurch könnte eine große Zahl von Adenom- bzw. Karzinomträgern so früh erkannt werden, dass eine Behandlung mit geringer Morbidität und geringeren Kosten möglich wird.

Das erfindungsgemäße Verfahren wird *in vitro* durchgeführt. Die Probe biologischen Materials, die zur Diagnose des Pankreasadenoms einem Patienten entnommen wird, ist bevorzugt eine Blutprobe oder eine Serumprobe, die Proteine enthält. Jedes verwendete Biomarker-Protein umfasst bevorzugt ein Molekül ganzer Länge oder ein Fragment davon, welches zum Nachweis von Anti-Biomarker-Antigen Autoantikörpern geeignet ist. Bevorzugt sind die Biomarker an einen Träger gebunden. Ein solcher fester Träger kann z.B. eine ELISA-Platte, ein magnetisches Kügelchen oder eine Blotfolie, z.B. eine Nitrozellulosefolie, sein. Die Biomarker-Proteine können direkt an den Träger gebunden, oder, z.B. über Antikörper, insbesondere Antikörper gegen einen mit Biomarker-Proteine verbundenen Tag, wie Glutathion-S-Transferase, an einen Träger gekoppelt werden. Diese Antikörper sind keine humanen Antikörper, um Kreuzreaktionen mit sekundären Antikörpern zu verhindern. In einer bevorzugten Ausführungsform der Erfindung wird die Bindung von Autoantikörpern an ihren Antigenen untersucht, indem man den Träger mit markierten anti-Immunglobulin-Antikörpern gegen Antikörper des Patients kontaktiert, und man die markierten Antikörper nachweist. Bevorzugt sind die anti-Immunglobulin-Antikörper mit einem Enzym, beispielsweise Alkalischer Phosphatase oder Meerrettich-Peroxidase, Biotin, einem radioaktiven Isotop oder einem Fluoreszenzfarbstoff, z.B. Fluoresceinisothiozyanat (FITC) oder Phycoerythrin (PE) markiert. Die Untersuchung kann in einem Blot, z.B. einem Dotblot oder einem Westernblot, einem ELISA (Enzyme linked immunosorbent assay), einem RIA (Radioimmunoassay), einer FACS (Fluorescence activated cell sorting)-Untersuchung oder auch in einem Flüssigphasendetektionssystem durchgeführt werden.

Ebenfalls wird die Verwendung eines Kits zur Durchführung des erfindungsgemäßen Verfahrens bereitgestellt. Bevorzugt umfasst dieses Kit Biomarker-Proteine und markierte anti-Immunglobulin-Antikörper. Jedes verwendete Biomarker-Protein umfasst bevorzugt ein Molekül ganzer Länge oder ein Fragment davon, welches zum Nachweis von Anti-Biomarker-Antigen Autoantikörpern geeignet ist. Die in dem Kit enthaltenen markierten Antikörper können mit einem Enzym, z.B. alkalischer Phosphatase oder Meerrettich-Peroxidase, Biotin, einem radioaktiven Isotop oder einem Fluoreszenzfarbstoff, z.B. FITC oder PE, markiert sein.

Schließlich betrifft die Erfindung noch die Verwendung von Autoantikörpern gegen die Biomarker-Proteine als diagnostischer Marker bei Patienten mit Pankreasadenom und somit die Verwendung eines hierin beschriebenen Kits zur Bestimmung von Autoantikörpern gegen die Biomarker-Proteine als diagnostischer Marker für die Überlebensprognose bei Patienten mit Pankreasadenom.

Die Erfindung wird anhand des folgenden Beispiels näher beschrieben.

### Beispiel 1:

Alle Seren wurden nach einer identischen Prozedur verarbeitet. Blutproben wurden bei Raumtemperatur für maximal 30 min gelagert, um eine Gerinnung zu ermöglichen, und anschließend bei 3000 x g für 10 min bei 4 °C zentrifugiert. Bis zur weiteren Verarbeitung wurden die Seren bei -180 °C asserviert. 25 Serumproben (10 Seren von Patienten mit Pankreastumoren, 5 Patienten mit einem Pankreasadenom und 10 gesunde Probanden) wurden mittels dem Human ProtoArrayTM v5.0 (Invitrogen) nach Empfehlungen des Herstellers beprobt und analysiert: Alle ProtoArrays wurden 20 min bei 4 °C äquilibriert und dann unter leichtem Schütteln mit Blocking-Puffer (1 % BSA, 0,5 mM DTT, 5 % Glycerin, 0,05 % Triton X-100 in PBS) für 1 h bei 4 °C inkubiert. Anschließend wurden die Arrays mit 10 µL Serum (1:500 Verdünnung) für 90 min bei 4 °C bedeckt und gewaschen (1 % BSA in 0,1 % Tween 20, PBS). Humane gebundene Antikörper wurden nach Inkubation für 90 min bei 4 °C mit Alexa Fluor nachgewiesen (647-Goat-anti-human IgG (Invitrogen), 1:2000 Verdünnung). Zur Quantifizierung der Signale diente ein Axon 4200AL Scanner (Molecular Devices) mit GenePix Pro 5.1 Bildanalyse-Software (Molecular Devices).

**Tabelle 1: Autoantikörper Herunterregulation in Pankreasadenomen im Vergleich zu gesunden Kontrollen**

| **Gen** | **NCBI ID** | **Beschreibung** | **P-Wert** |
|---|---|---|---|
| KCNAB1 | NM_172159.2 | Potassium voltage-gated channel, shaker-related subfamily, beta member 1 (KCNAB1), transcript variant 3 | 0,00366 |
| C1ORF77 | BC002733.2 | Chromosome 1 open reading frame 77 (C1orf77) | 0,01698 |
| SPSB2 | NM_032641.1 | SplA/ryanodine receptor domain and SOCS box containing 2 (SPSB2) | 0,01865 |
| MED9 | NM_018019.1 | Mediator complex subunit 9 (MED9) | 0,01865 |
| STK40 | NM_032017.1 | Serine/threonine kinase 40 (STK40) | 0,02198 |
| ITGB3BP | NM_014288.2 | Centromere protein R | 0,04196 |
| SCEL | BC047536.1 | Sciellin (SCEL) | 0,04196 |
| PDPK1 | NM_002613.3 | 3-phosphoinositide dependent protein kinase-1 (PDPK1), transcript variant 1 | 0,04196 |
| DUSP15 | BC056911.1 | Dual specificity phosphatase 15 (DUSP15) | 0,04196 |
| MED19 | NM_153450.1 | Mediator complex subunit 19 (MED19) | 0,04196 |

**Tabelle 2: Autoantikörper Hochregulation in Pankreasadenomen im Vergleich zu gesunden Kontrollen**

| **Gen** | **NCBI ID** | **Beschreibung** | **P-Wert** |
|---|---|---|---|
| NAP1L3 | BC34954.2 | Nucleosome assembly protein 1-like 3 (NAP1L3) | 0,01698 |
| TMOD3 | NM_014547.3 | Tropomodulin-3 | 0,01698 |
| CSPP1 | NM_024790.2 | Centrosome and spindle pole associated protein 1 (CSPP1), transcript variant 2 | 0,01698 |
| TMOD2 | BC036184.1 | Tropomodulin-2 | 0,01865 |
| TMOD2 | NM_014548.2 | Tropomodulin 2 (neuronal) (TMOD2) | 0,01865 |
| INPP5A | BC096280.2 | Type I inositol-1,4,5-trisphosphate 5-phosphatase | 0,02198 |
| IGHG1 | BC014667.1 | Immunoglobulin heavy constant gamma 1 (G1m marker) (IGHG1) | 0,02198 |

## Patentansprüche

1. Verfahren zur *in vitro* Diagnose eines Pankreasadenoms, mit den Schritten:
(a) Inkubieren einer einem Individuum entnommenen Antikörper enthaltenden Probe mit wenigstens einem von einem der Gene ausgewählt aus einer ersten Gruppe von Genen bestehend aus KCNAB1, C1ORF77, SPSB2, MED9, STK40, ITGB3BP, SCEL, PDPK1, DUSP15 und MED19 codierten Protein oder Proteinfragments;
(b) Bestimmen der an das wenigstens eine Protein oder das Proteinfragment gebundenen Antikörpermenge; und
(c) Vergleichen der aus der Probe an das wenigstens eine Protein oder Proteinfragment gebundenen Antikörpermenge mit der bei gesunden Individuen im Durchschnitt gegen das die wenigstens eine Protein oder Proteinfragment vorliegenden Antikörpermenge,
wobei eine niedrigere Menge des bestimmten gegen das wenigstens eine von der ersten Gruppe von Genen codierte Protein oder Proteinfragment gerichteten Antikörpers das Vorliegen eines Pankreasadenoms anzeigt.

2. Verfahren nach Anspruch 1, **dadurch gekennzeichnet, dass** das Verfahren weiterhin die Schritte umfasst:
(a) Inkubieren der Probe mit wenigstens einem von einem der Gene ausgewählt aus einer zweiten Gruppe von Genen bestehend aus NAP1L3, TMOD3, CSPP1, TMOD2, INPP5A und IGHG1 codierten Protein oder Proteinfragments,
(b) Bestimmen der an das wenigstens eine Protein oder das Proteinfragment gebundenen Antikörpermenge; und
(c) Vergleichen der aus der Probe an das wenigstens eine Protein oder Proteinfragment gebundenen Antikörpermenge mit der bei gesunden Individuen im Durchschnitt gegen das die wenigstens eine Protein oder Proteinfragment vorliegenden Antikörpermenge,
wobei eine höhere Menge des bestimmten gegen das wenigstens eine von der zweiten Gruppe von Genen codierte Protein oder Proteinfragment gerichteten Antikörpers, das Vorliegen eines Pankreasadenoms anzeigt.

3. Verfahren nach Anspruch 2, **dadurch gekennzeichnet, dass** das wenigstens eine Protein oder Proteinfragment von TMOD3 oder INPP5A codiert ist.

4. Verfahren nach einem der Ansprüche 1 bis 3, **dadurch gekennzeichnet, dass** die Probe Blut oder Serum ist.

5. Verfahren nach einem der vorhergehenden Ansprüche, **dadurch gekennzeichnet, dass** das wenigstens eine Protein oder Proteinfragment an einen Träger gebunden ist.

6. Verfahren nach einem der vorhergehenden Ansprüche, **dadurch gekennzeichnet, dass** das Bestimmen der an das wenigstens eine Protein oder Proteinfragment gebundenen Antikörpermenge mittels markierter anti-human-Immunglobulin-Antikörper erfolgt.

7. Verfahren nach Anspruch 6, **dadurch gekennzeichnet, dass** die anti-human-Immunglobulin-Antikörper mit einem Enzym, Biotin, einem radioaktiven Isotop oder einem Fluoreszenzfarbstoff markiert sind.

8. Verwendung eines Kits zum Durchführen des Verfahrens nach einem der vorhergehenden Ansprüche, **gekennzeichnet durch** wenigstens ein von einem der Gene ausgewählt aus einer ersten Gruppe von Genen bestehend aus KCNAB1, C1ORF77, SPSB2, MED9, STK40, ITGB3BP, SCEL, PDPK1, DUSP15 und MED19 und gegebenenfalls wenigstens einem von einem der Gene ausgewählt aus einer zweiten Gruppe von Genen bestehend aus NAP1L3, TMOD3, CSPP1, TMOD2, INPP5A und IGHG1 codierten Protein oder Proteinfragment; und wenigstens einen markierten anti-human-Immunglobulin-Antikörper.

9. Verwendung nach Anspruch 8, **dadurch gekennzeichnet, dass** das wenigstens eine Protein oder Proteinfragment von TMOD3 oder INPP5A codiert ist.

10. Verwendung nach Anspruch 8 oder 9, **dadurch gekennzeichnet, dass** das Protein oder Proteinfragment an einem Träger immobilisiert ist.

11. Verwendung nach einem der Ansprüche 8 bis 10, **dadurch gekennzeichnet, dass** der anti-human-Immunglobulin-Antikörper mit einem Enzym, Biotin, einem radioaktiven Isotop oder einem Fluoreszenzfarbstoff markiert ist.

12. Verwendung wenigstens eines Autoantikörpers gerichtet gegen ein Protein oder Proteinfragment, das von einem Gen codiert wird, ausgewählt aus der Gruppe bestehend aus KCNAB1, C1ORF77, SPSB2, MED9, STK40, ITGB3BP, SCEL, PDPK1, DUSP15 und MED 19 als diagnostischer Marker für die Diagnose eines Pankreasadenoms *in vitro.*

## Claims

1. A method for *in vitro* diagnosis of a pancreatic adenoma comprising the steps of:
(a) incubating an antibody containing sample derived from an individual with at least one protein or protein fragment encoded by any one of the genes selected from a first group of genes consisting of KCNAB1, C1ORF77, SPSB2, MED9, STK40, ITGB3BP, SCEL, PDPK1, DUSP15 and MED19;
(b) determining the amount of antibody bound to the at least one protein or protein fragment; and
(c) comparing the amount of antibody bound to the at least one protein or protein fragment from the sample to the average antibody amount against the at least one protein or protein fragment present in healthy individuals,
wherein a lower amount of the particular antibody directed against the at least one protein or protein fragment encoded by the first group of genes indicates the presence of a pancreatic adenoma.

2. The method according to claim 1, **characterized in that** the method further comprises the steps of:
(a) incubating the sample with at least one protein or protein fragment encoded by any one of the genes selected from a second group of genes consisting of NAP1L3, TMOD3, CSPP1, TMOD2, INPP5A and IGHG1,
(b) determining the amount of antibody bound to the at least one protein or protein fragment; and
(c) comparing the amount of antibody bound to the at least one protein or protein fragment from the sample to the average antibody amount against the at least one protein or protein fragment present in healthy individuals,
wherein a higher amount of the particular antibody directed against the at least one protein or protein fragment encoded by the second group of genes indicates the presence of a pancreatic adenoma.

3. The method according to claim 2, **characterized in that** the at least one protein or protein fragment is encoded by TMOD3 or INPP5A.

4. The method according to any one of claims 1 to 3, **characterized in that** the sample is blood or serum.

5. The method according to any one of the preceding claims, **characterized in that** the at least one protein or protein fragment is bound to a carrier.

6. The method according to any one of the preceding claims, **characterized in that** the determination of the amount of antibody bound to the at least one protein or protein fragment is performed by means of labeled anti-human immunoglobulin antibodies.

7. The method according to claim 6, **characterized in that** the anti-human immunoglobulin antibodies are labeled with an enzyme, biotin, a radioactive isotope or a fluorescence dye.

8. Use of a kit for carrying out the method according to any one of the preceding claims, **characterized by** at least one protein or protein fragment encoded by any one of the genes selected from a first group of genes consisting of KCNAB1, C1ORF77, SPSB2, MED9, STK40, ITGB3BP, SCEL, PDPK1, DUSP15 and MED19 and optionally by at least one protein or protein fragment encoded by any one of the genes selected from a second group of genes consisting of NAP1L3, TMOD3, CSPP1, TMOD2, INPP5A and IGHG1; and at least one labeled anti-human immunoglobulin antibody.

9. The use according to claim 8, **characterized in that** the at least one protein or protein fragment is encoded by TMOD3 or INPP5A.

10. The use according to claim 8 or 9, **characterized in that** the protein or protein fragment is immobilized to a carrier.

11. The use according to any one of claims 8 to 10, **characterized in that** the anti-human immunoglobulin antibody is labeled with an enzyme, biotin, a radioactive isotope or a fluorescence dye.

12. Use of at least one auto-antibody directed against a protein or protein fragment encoded by a gene selected from the group consisting of KCNAB1, C1ORF77, SPSB2, MED9, STK40, ITGB3BP, SCEL, PDPK1, DUSP15 and MED19 as diagnostic marker for the diagnosis of a pancreatic adenoma *in vitro.*

## Revendications

1. Procédé pour le diagnostic *in vitro* d'un adénome du pancréas, avec les étapes:
(a) incuber un échantillon contenant des anticorps prélevé auprès d'un individu avec au moins une protéine ou au moins un fragment de protéine codé(e) par l'un des gènes choisis dans un premier groupe de gènes consistant en KCNAB1, C1ORF77, SPSB2, MED9, STK40, ITGB3BP, SCEL, PDPK1, DUSP15 et MED19;
(b) déterminer la quantité d'anticorps liée à la au moins une protéine ou au au moins un fragment de protéine; et
(c) comparer la quantité d'anticorps liée à la au moins une protéine ou au au moins un fragment de protéine provenant de l'échantillon avec la quantité d'anticorps contre la au moins une protéine ou le au moins un fragment de protéine présente en moyenne chez des individus en bonne santé,
où une plus faible quantité de l'anticorps déterminé dirigé contre la au moins une protéine ou le au moins un fragment de protéine codé(e) par le premier groupe de gènes indique la présence d'un adénome du pancréas.

2. Procédé selon la revendication 1, **caractérisé en ce que** le procédé comprend en outre les étapes:
(a) incuber l'échantillon avec au moins une protéine ou au moins un fragment de protéine codé(e) par l'un des gènes choisis dans un deuxième groupe de gènes consistant en NAP1L3, TMOD3, CSPP1, TMOD2, INPP5A et IGHG1;
(b) déterminer la quantité d'anticorps liée à la au moins une protéine ou au au moins un fragment de protéine; et
(c) comparer la quantité d'anticorps liée à la au moins une protéine ou au au moins un fragment de protéine provenant de l'échantillon avec la quantité d'anticorps contre la au moins une protéine ou le au moins un fragment de protéine présente en moyenne chez des individus en bonne santé,
où une plus grande quantité de l'anticorps déterminé dirigé contre la au moins une protéine ou le au moins un fragment de protéine codé(e) par le deuxième groupe de gènes indique la présence d'un adénome du pancréas.

3. Procédé selon la revendication 2, **caractérisé en ce que** la au moins une protéine ou le au moins un fragment de protéine est codé(e) par TMOD3 ou INPP5A.

4. Procédé selon l'une des revendications 1 à 3, **caractérisé en ce que** l'échantillon est du sang ou du sérum.

5. Procédé selon l'une des revendications précédentes, **caractérisé en ce que** la au moins une protéine ou le au moins un fragment de protéine est lié(e) à un support.

6. Procédé selon l'une des revendications précédentes, **caractérisé en ce que** la détermination de la quantité d'anticorps liée à la au moins une protéine ou au au moins un fragment de protéine a lieu au moyen d'anticorps anti-immunoglobulines humaines marqués.

7. Procédé selon la revendication 6, **caractérisé en ce que** les anticorps anti-immunoglobulines humaines sont marqués avec une enzyme, la biotine, un isotope radioactif ou un colorant fluorescent.

8. Utilisation d'un kit pour la mise en oeuvre du procédé selon l'une des revendications précédentes, **caractérisée par** au moins une protéine ou au moins un fragment de protéine codé(e) par l'un des gènes choisis dans un premier groupe de gènes consistant en KCNAB1, C1ORF77, SPSB2, MED9, STK40, ITGB3BP, SCEL, PDPK1, DUSP15 et MED 19 et éventuellement au moins une protéine ou au moins un fragment de protéine codé(e) par l'un des gènes choisis dans un deuxième groupe de gènes consistant en NAP1L3, TMOD3, CSPP1, TMOD2, INPP5A et IGHG1; et au moins un anticorps anti-immunoglobulines humaines marqué.

9. Utilisation selon la revendication 8, **caractérisée en ce que** la au moins une protéine ou le au moins un fragment de protéine est codé(e) par TMOD3 ou INPP5A.

10. Utilisation selon la revendication 8 ou 9, **caractérisée en ce que** la protéine ou le fragment de protéine est immobilisé(e) sur un support.

11. Utilisation selon l'une des revendications 8 à 10, **caractérisée en ce que** l'anticorps anti-immunoglobulines humaines est marqué avec une enzyme, la biotine, un isotope radioactif ou un colorant fluorescent.

12. Utilisation d'au moins un autoanticorps dirigé contre une protéine ou un fragment de protéine qui est codé(e) par un gène choisi dans le groupe consistant en KCNAB1, C1ORF77, SPSB2, MED9, STK40, ITGB3BP, SCEL, PDPK1, DUSP15 et MED19 comme marqueur diagnostique pour le diagnostic d'un adénome du pancréas *in vitro.*
